# EUROPEAN PATENT APPLICATION

(11) **EP 2 977 059 A1**
(43) Date of publication of application: **27.01.2016**
(21) Application number: 14306187.7
(22) Date of filing: 22.07.2014
(51) Int. Cl.: A61K 39/00

(54) **Immunogenic polypeptide composed of HLA-B7 restricted tumor antigen-derived optimized cryptic peptides, and uses thereof**

(71) Applicant: Vaxon Biotech, 75749 Paris Cedex 15 (FR)
(72) Inventor: Gallou, Catherine, 91440 BURES SUR YVETTE (FR); Menez-Jamet, Jeanne, 92120 MONTROUGE (FR)
(74) Representative: Marcadé, Véronique

(57) **Abstract**

The invention pertains to an optimized chimeric polypeptide for use in HLA-B7 cancer patients, which comprises four optimized peptides derived from cryptic tumor epitopes (CEA, TERT, MAGE and HER-2/neu) to enhance their immunogenicity.

## Description

The present invention pertains to the field of anti-cancer vaccines. More particularly, the invention relates to an optimized chimeric polypeptide for use in HLA-B7 cancer patients, which comprises four optimized peptides derived from cryptic tumor epitopes to enhance their immunogenicity.

Antitumor vaccines currently in development take many forms, including free peptides, recombinant proteins, dendritic cells loaded with peptides or tumor lysates, and DNA. Although peptide-based vaccines are very attractive over other forms in terms of feasibility, many studies with vaccines targeting dominant tumor peptides were found to elicit only weak immunological and clinical responses, with strong inter-patient variability. This was the case of several peptide-vaccines derived from gp100 tested in several clinical studies, the MUC1 derived BLP25 peptide tested in a randomized phase II trial and the HER-2/neu derived E75 peptide tested in a very large number of phase I and phase II clinical studies (Butts et al, 2005; Peoples et al, 2008; Rosenberg et al, 2004).

Optimized cryptic peptides induce antitumor immunity more efficiently than dominant peptides in transgenic mice model (Gross et al, 2004) and two vaccines based on this technology are currently in clinical development:
- Vx-001, a TERT-derived mono-epitope peptide designated to NSCLC cancer HLA-A2 patients, is in phase IIb and
- Vx-006, a three-epitopes polypeptide which targets TERT, MAGE-A and HER-2/neu universal tumor antigens also designated to HLA-A2 patients, is in phase I.

Approaches eliciting CTL responses to multiple antigens using polypeptide-based vaccine have several advantages. In particular, expression of at least one target antigen should be sufficient to trigger killing of tumor cells by vaccine-induced CTLs, and tumor cells are unlikely to lose all the target antigens simultaneously, especially when these antigens are essential for cell survival and tumor growth. This approach can elicit strong immune responses (Oukka et al, 1996) and increase the proportion of patients likely to benefit from the vaccine. Moreover, broad-spectrum cancer vaccines should target universal tumor antigens, such as TERT, HER-2/neu, MUC-1, CEA, EphA2 and MAGE-A, which are over-expressed by a wide variety of tumors (Minev et al, 2000; Ofuji et al, 1998; Ogata et al, 1992; Reese & Slamon, 1997; Slamon et al, 1987; Van den Eynde & van der Bruggen, 1997; Vonderheide et al, 1999). Most of these antigens are involved in tumor cell survival and tumorigenicity, and their down-regulation to escape the immune response may therefore have deleterious effect on tumor growth.

To increase the number of patients who can benefit from such products, the inventors have developed a polypeptide-based vaccine, named Vbx-016, designated to HLA-B7 expressing patients, which targets four widely expressed tumor antigens: TERT, HER-2/neu, MAGE and CEA. The four optimized cryptic peptides (Table 1) that compose the Vbx-016, were already described (WO2008/010098, WO2010/143010). Each of the four peptides was shown to elicit an antitumor *response in vivo* and *in vitro* (WO2008/010098, WO2010/143010), both against the optimized peptide and against the native cognate peptide naturally expressed by tumour cells (table 1). Interestingly, CTLs elicited by MAGE-A_{273V6L9} targeted six MAGE-A antigens (-A1, -A2, -A3, -A4, -A6, and - A12). Indeed, specific CTLs induced using the MAGE-A_{273V6L9} are able to recognize cells loaded with MAGE-A₂₇₃A6 (included in MAGE-A1 and MAGE-A4), MAGE-A₂₇₃I6 (included in MAGE-A2 and MAGE-A6) and MAGE-A₂₇₃V6 (included in MAGE-A3 and MAGE-A12).

**Table 1: Native and optimized epitope sequences**

| **Name native optimized peptide** | **Antigen_{Position}** | **Modification** | **Sequence** | **size** | **Seq ID** |
|---|---|---|---|---|---|
| 7C1 | CEA₁₈₈ | | SPRLQLSNG | 9 | 1 |
| 7C1M | CEA₁₈₈ | L9 | SPRLQLSNL | 9 | 7 |
| 7HN3 | HER-2/neu₂₄₆ | | GPKHSDCLA | 9 | 2 |
| 7HN3M | HER-2/neu₂₄₆ | A1 | APKHSDCLA | 9 | 8 |
| 7T5 | TERT₄₄₄ | | DPRRLVQLL | 9 | 3 |
| 7T5M | TERT₄₄₄ | A1 | APRRLVQLL | 9 | 9 |
| 7M1A6 | MAGE-A₂₇₃ A6 | | GPRALAETS | 9 | 4 |
| 7M1V6 | MAGE-A₂₇₃ V6 | | GPRALIETS | 9 | 5 |
| 7M1I6 | MAGE-A₂₇₃I6 | | GPRALVETS | 9 | 6 |
| 7M1M | MAGE-A₂₇₃V6 | L9 | GPRALVETL | 9 | 10 |

Classically, polypeptide-based vaccines are emulsified with an adjuvant such as Montanide ISA51VG (Seppic, Castres, France) before subcutaneous injection. After injection, the polypeptide is internalized into professional Antigen Presenting Cells (APC), especially Langerhans cells present locally in the skin. The polypeptide is then processed by the proteasome in the Golgi apparatus and the epitopes are presented at the cell surface in association with HLA molecules. To ensure the efficient presentation of each epitope of the polypeptide, it is mandatory to test if each junction is correctly processed by the proteasome. Moreover, the inventors have previously described, with Vx006 three-epitopes polypeptide, that the order of the epitopes in the sequence of a polyepitopic vaccine is crucial for each epitope processing and finally for obtaining an immune response against all the corresponding epitopes (WO2007/073768).

To avoid testing the 24 putative arrangements, the optimal organization of the four optimized peptides in the polypeptide was then determined in two steps: (i) test of each junctional sequences; and (ii) test of the corresponding complete polypeptide.

Each potential junction was thus tested. Twelve possible dipeptides presented in table 2 were then *tested in vivo* in HLA-B*0702 transgenic mice and *in vitro* in healthy donor PBMCs culture for:
- the capacity of the dipeptide to be efficiently processed and to induce specific CTLs able to recognize cells loaded with each of the optimized epitopes,
- the capacity of induced CTLs to recognize the cognate native peptides corresponding to the sequence naturally present at the tumor cell surface.

**Table 2. Dipeptides sequences tested in transgenic mice**

| **Name** | **Sequence** | **Size (AA)** | **SEQ ID No:** |
|---|---|---|---|
| 7C1HN3M | SPRLQLSNLAPKHSDCLA | 18 | 11 |
| 7HN3C1M | APKHSDCLASPRLQLSNL | 18 | 12 |
| 7C1T5M | SPRLQLSNLAPRRLVQLL | 18 | 13 |
| 7T5C1M | APRRLVQLLSPRLQLSNL | 18 | 14 |
| 7C1M1M | SPRLQLSNLGPRALVETL | 18 | 15 |
| 7M1C1M | GPRALVETLSPRLQLSNL | 18 | 16 |
| 7HN3T5M | APKHSDCLAAPRRLVQLL | 18 | 17 |
| 7T5HN3M | APRRLVQLLAPKHSDCLA | 18 | 18 |
| 7HN3M1M | APKHSDCLAGPRALVETL | 18 | 19 |
| 7M1HN3M | GPRALVETLAPKHSDCLA | 18 | 20 |
| 7T5M1M | APRRLVQLLGPRALVETL | 18 | 21 |
| 7M1T5M | GPRALVETLAPRRLVQLL | 18 | 22 |

First of all, each dipeptide sequence was submitted to the two main *in silico* proteasome cleavage prediction softwares, MAPPP (developed by Jörg Hakenberg and Hans-Joachim Mollenkopf at the Max-Planck-Institute for Infection Biology, available on the world wide web) and PAProC (Prediction Algorithm for Proteasomal Cleavages, also available on the world wide web) which predict the probability of processing of each epitope.

According to PAProC, none of the dipeptide should be processed correctly by the proteasome (predicted cleavage sites are represented by dashes, table 3), except the 7T5HN3M that could produce both epitopes (cleavage site with a double dash).

**Table 3: proteasome cleavage prediction for dipeptides (PaProc)**

| **Name** | **Cleavage sites prediction** | **SEQ ID No:** |
|---|---|---|
| 7C1HN3M | SPRLQL \| SNLAPKH \| S \| DCLA | 11 |
| 7HN3C1M | APKHSDC \| LASPRL \| QLSNL | 12 |
| 7C1T5M | SPRLQL \| SNLAPRR \| LVQLL | 13 |
| 7T5C1M | APRRLV \| QLLS \| PRL \| QLSNL | 14 |
| 7C1M1M | SPRLQL \| SNLGPRALVETL | 15 |
| 7M1C1M | GPRALVE \| TL \| \| S \| P \| RL \| QLSNL | 16 |
| 7HN3T5M | APKHSDC \| LAAPRR LVQLL | 17 |
| 7T5HN3M | APRRLV \| QLLAPKHS \| DCLA | 18 |
| 7HN3M1M | APKHSDC \| LAGPRALVETL | 19 |
| 7M1HN3M | GPRALVE \| TLAP \| KHS \| DCLA | 20 |
| 7T5M1M | APRRLV \| QLLGPRALVETL | 21 |
| 7M1T5M | GPRALVE \| TLAP \| RRLVQLL | 22 |

According to MAPPP prediction model, many combinations should allow the processing of the four epitopes (data not shown). The predicted best sequence that should ensure the efficient processing of each epitope by the proteasome is CEA_{188L9}/ MAGE_{273L9}/TERT_{444A1}/ HER-2/neu_{246A1}.

Dipeptides were then tested *in vivo* in HLA-B*0702 transgenic mice. As described in example 1, combinations 7C1HN3M, 7C1T5M, 7T5HN3M, 7M1HN3M and 7T5M1M gave better results than the corresponding inverted sequences, in terms of number of responding mice. 7C1M1M and 7M1C1M gave the same results.

Based on these *in vivo* experimental data, the following theoretical optimal polypeptide was then designed: CEA_{188L9}/TERT_{444A1}/MAGE_{273L9}/HER-2/neu_{246A1}.

In order to assess whether the epitopes are efficiently processed in the quadripeptide, the corresponding polypeptide SPRLQLSNLAPRRLVQLLGPRALVETLAPKHSDCLA (SEQ **ID** No: 23) was then tested in HLA-B*0702 transgenic mice for its capacity to induce specific CTLs recognizing the four optimized epitopes and the four cognate native peptides which are naturally present at the surface of tumor cells. As described in example 2, after two vaccinations with the polypeptide, all mice responded to at least one native peptide and a large majority of vaccinated mice responded to two or more cognate native peptides confirming that the polypeptide is efficiently processed by the proteasome. Importantly, all the native peptides were recognized at least once in one vaccinated mouse.

Interestingly, this sequence is different from the sequence selected by the proteasome MAPPP cleavage prediction model, since the 7M1T5M was predicted to not be processed correctly, contrary to what happens *in vivo.*

In order to confirm the results obtained in HLA-B*0702 transgenic mice *in vitro* in humans, each junction of the defined polypeptide (CEA_{188L9} /TERT_{444A1}, TERT_{444A1}/MAGE_{273L9} and MAGE_{273L9}/HER-2/ne_{U246A1}) was tested independently *in vitro* in a PBMC culture from a healthy human donor. Results described in example 3 show that each dipeptide was efficiently processed *in vitro* in humans and that specific CTLs induced were able to recognize cells loaded either with the optimized or with the cognate native peptide.

Finally, the polypeptide SPRLQLSNLAPRRLVQLLGPRALVETLAPKHSDCLA (SEQ **ID** No: 23) was tested in a PBMC culture from one HLA-B*0702 healthy donor (example 4), confirming the capacity of the polypeptide to generate a polyspecific CTLs immune response. Importantly, several native peptides were recognized. These results confirm that the polypeptide is processed efficiently by the proteasome and that the induced immune response is polyspecific.

A first aspect of the invention is hence a polypeptide which comprises the sequence SPRLQLSNLXXXAPRRLVQLLXXXGPRALVETLXXXAPKHSDCLA (Seq ID No: 24). In this sequence, the CEA_{188L9}, TERT_{444A1}, MAGE_{273L9} and HER-2/neu_{246A1} epitopes are separated by spacers XXX, in which each X is (independently from each other) any amino acid or none. The polypeptide is hence at least 36-aminoacids long; its length can be increased by the addition of spacers between the epitopes, and/or by the addition of signals, at its N-terminal and/or C-terminal extremities, which favor its processing. In particular, the polypeptide according to the invention can further comprise an endoplasmic reticulum-translocating signal sequence at its N-terminal extremity. Several endoplasmic reticulum-translocating signal sequences have been described in the scientific literature and can be used in the context of the invention. For example, the Ig kappa-chain signal sequence (Ishioka et al, 1999), and the E3/19-kD protein signal sequence (Anderson et al, 1991) can be added at the N-terminal extremity of the peptides according to the invention. Alternatively or in addition, the polypeptide according to the invention can further comprise ubiquitin at its C-terminal extremity, since ubiquitination of proteins results in increased proteolysis.

In a preferred embodiment of the polypeptide according to the invention, the four epitopes are directly bound to each other without the use of any spacer (X=none for each position). Hence, the polypeptide comprises the sequence SPRLQLSNLAPRRLVQLLGPRALVETLAPKHSDCLA (Seq ID No: 23). In the absence of ubiquitin and ER-translocating signal, the polypeptide hence consists of the polypeptide illustrated in the examples below (SPRLQLSNLAPRRLVQLLGPRALVETLAPKHSDCLA, Seq ID No: 23).

In the polypeptides according to the invention, the amino acids can be either L- or D-amino acids.

A polypeptide according to the invention induces a specific CD8⁺ T cells response against at least one and preferably at least two peptides selected amongst CEA₁₈₈, TERT₄₄₄, MAGE₂₇₃ and HER-2/neu₂₄₆ cognate native peptides, in a majority of HLA-B*0702 transgenic mice vaccinated with said polypeptide.

A polypeptide according to the invention also induces a specific CD8⁺ T cells response against at least one and preferably at least two peptides selected amongst CEA₁₈₈, TERT₄₄₄, MAGE₂₇₃ and HER-2/neu₂₄₆ native peptides in an *in vitro* assay with human PBMC from healthy HLA-B*0702 donors

In order to control that the polypeptide of SEQ ID No: 23 is correctly processed and has a good immunogenicity, the inventors have also demonstrated that:
- each native peptide is recognized at least once in a HLA-B*0702 transgenic mice;
- the polypeptide of SEQ ID No: 23 induces a specific CD8⁺ T cells response against at least one and usually two or more peptides selected amongst CEA₁₈₈, TERT₄₄₄, MAGE₂₇₃ and HER-2/neu₂₄₆ cognate native peptides in an *in vitro* assay with human PBMC from healthy HLA-B*0702 donors; and
- these specific responses are obtained with PBMC from two or more healthy HLA-B*0702 donors and each optimized peptide is recognized at least once in an *in vitro* assay with human PBMC from a HLA-B*0702 healthy donor.

In a more preferred embodiment, the polypeptide according to the invention exhibits all the above properties, which can easily be tested by the skilled artisan, using the protocols and assays described in the experimental part below.

Another aspect of the invention is an isolated dendritic cell loaded with a polypeptide as above-described. In the present context "isolated" means that said dendritic cell is outside the body of the patient. The cell is preferably loaded *ex vivo.* For example, the dendritic cell can be loaded with the polypeptide by the technique described by Vonderheide *et al.* (Vonderheide et al, 2004).

The invention also pertains to a complex comprising a peptide delivery vector and a polypeptide as described above. Examples of peptide delivery vectors that can be used according to the invention are cell-penetrating peptides such as those described, for example, in the patent applications published as WO2013/150338, EP1795539 and WO2014/053629, bacterial toxins such as the adenylate cyclase of *B. pertussis* (Fayolle et al, 1999), the diphtheria toxin (Fayolle et al, 1999), the anthrax toxin (Doling et al, 1999), the B subunit of shiga toxin (Haicheur et al, 2000) and other vectors such as the bee venom PLA2 (Babon et al, 2005), liposomes, virosomes (Bungener et al, 2002) and the like.

The invention also concerns a pharmaceutical composition comprising a polypeptide and/or an engineered dendritic cell and/or a complex as described above, as well as a pharmaceutically acceptable carrier. In particular, polypeptides, dendritic cells and complexes according to the invention can be used for the preparation of an immunogenic composition for anti-cancer immunotherapy. These compositions are particularly useful for immunoherapy of tumors which express at least one antigen selected in the group consisting of the MAGE-A family, the HER family, CEA and TERT, especially for treating HLA-B*0702 individuals.

The present invention can be used to treat (or prevent relapse of) virtually any type of cancers, such as adrenal cortical cancer, colorectal cancer, biliary tract carcinoma, bladder cancer, bone cancers, brain and central nervous system cancers, breast cancer, cervical cancer, endometrial cancers, oesophagus cancer, gastric cancer, gastrointestinal carcinoid tumors, Hodgkin's disease, non-Hodgkin's lymphoma, Kaposi's sarcoma, kidney cancer, Head and Neck cancer, liver cancers, lung cancers mesothelioma, ovarian cancer, pancreatic cancer, penile cancer, pituitary cancer, prostate cancer, retinoblastoma, rhabdomyosarcoma, , skin cancer (e.g. melanoma, non-melanoma skin cancer), testicular cancers, thymus cancer, thyroid cancers, vaginal cancer, vulvar cancer, and uterine cancer.

Cancer vaccination or treatment methods, comprising a step of emulsifying the polypeptide with an adjuvant and administering a polypeptide according to the invention *in vivo* to a patient in need thereof, are also part of the invention, as well as vaccination or treatment methods comprising a step of administering engineered dendritic cells or complexes as described above to an individual.

Another aspect of the present invention is a kit of parts comprising at least one dose of polypeptide (or complexes) as described above and at least one dose of adjuvant. Immunological adjuvants are substances which are added to vaccine formulations to increase their efficacy. Adjuvants can increase the magnitude and duration of the immune response induced by vaccination. Preferred examples of adjuvants which can be used in the kits according to the invention are those derived from the Incomplete Freund's adjuvant (IFA). Vaccine formulations with these adjuvants are water-in-oil (W/O) emulsions. Non-limitative examples of IFA-type detoxified adjuvants which can be used to for emulsions with the peptide according to the present invention include the mineral oil-based Montanide® ISA 51 and the squalene-based Montanide® ISA 720 (SEPPIC, France).

Another kit of parts according to the present invention comprises at least two doses of polypeptide or complexes as described above. The peptide can be already formulated with the adjuvant or not.

Since cancer vaccination necessitates several stimulations to be fully efficient, a kit of parts according to the present invention can comprise 3 to 50, preferably 6 to 20 doses of polypeptide as above-described.

According to a preferred embodiment of the kits described above, each dose of polypeptide comprises between 0.5 and 10 mg of polypeptide.

The invention is further illustrated by the following examples.

### EXAMPLES

The examples have been performed using the following materials and methods:
***Transgenic Mice.*** The HLA-B7 H-2 class-I knockout mice were previously described (Rohrlich et al, 2003) and kindly provided by F. Lemonnier (Institut Pasteur, Paris, France).
***Cells***. HLA-B*0702 transfected human T2-B7 cells were previously described (Rohrlich et al, 2003). Cells were grown in penicillin streptomycin FCS 20% supplemented RPMI1640 culture medium.
***Peptides and Plasmids.*** Peptides were synthesized either by Millegen (Labège, France) or Eurogentec (Seraing, Belgique).
***CTL Induction in vivo in HLA-B*0702 Transgenic Mice.*** HLA-B*0702 transgenic mice were vaccinated subcutaneously at the base of the tail with 200µg of optimized dipeptides or 400µg of Vbx-016 in association with the HV core T13L helper (150 µg) helper peptide and emulsified in Incomplete Freund Adjuvant (IFA) or Montanide ISA51VG (Seppic, Castres, France) twice at two weeks interval.

Seven days after the last vaccination, spleens were removed and T cells were isolated by Ficoll centrifugation and tested *ex vivo* for the recognition of 10µM of either the native or optimized peptides. Positive control was Concanavalin A and negative control was medium alone. All conditions were tested in quadriplicates. IFNγ producing T cells was quantified by ELISpot using the Diaclone Kit Murine IFNγ ELISpot.

An immune response was considered positive when there was 1) more than 10 spots difference/10⁶ cells between the negative control and the tested peptide and 2) a statistically significant difference between these two groups with T Test (p<0.05).

***Generation of** CTL from human PBMC.* PBMC were collected by leukapheresis from healthy HLA-B*0702 volunteers. Dendritic cells (DC) were produced from adherent cells cultured for seven days with 500 IU/ml GM-CSF and 500 IU/ml IL-4, in complete synthetic medium (AIMV). On day 6, DCs were pulsed with 10 µM of polypeptides overnight. On day 7, CD8+ cells were purified by negative selection with CD8 Dynabeads Untouched Human CD8. CD8+ cells (2x10⁵) + CD8- cells (6x10⁴) were stimulated with 3x10⁴ peptide-pulsed DC in AIMV medium supplemented with 1000 IU/ml IL-6 and 5 IU/ml IL-12, 1µg/ml anti-CD40 and 500 IU/ml IFNγ in round-bottomed 96-well plates.

From day 7, cultures were restimulated weekly with peptide-loaded DC in the presence of 20 IU/ml IL-2 and 10 ng/ml IL-7, 1 µg/ml anti-CD40 and and 500 IU/ml IFNγ.

After the third restimulation, CD8 cells were maintained in AIMV supplemented with 20 IU/ml IL-2 during 7 days. Cultures were starved during one night with AIMV and then were tested in an IFNγ ELISpot assay.

***IFNγ** ELISpot Assay.* For one culture of 96 wells, four pools of 24 wells were collected, CD8 cells were counted and 50 000 CD8 per well were dispatched on the ELISpot plate with 25 000 T2B7 loaded eiher with 10µM of each native monopeptide or with 10µM of modified monopeptides composing the polypeptide. Eight replicates of each condition were performed. Positive control was Phytohaemagglutinin A and negative control was T2B7 loaded with an irrelevant peptide not able to bind HLA-B7 molecules.

### Example 1: Evaluation of T cell immune response generated in HLA-B*0702 transgenic mice after two vaccinations with all different combinations of dipeptides

Each dipeptide was used to vaccinate HLA-B*0702 transgenic mice twice at 2 weeks interval and tested for its capacity to induce an immune response against both optimized peptides and their cognate native counterparts. Dipeptides are described in table 2. In each experiment, the best combination is highlighted in grey. 7C1HN3M, 7C1T5M, 7T5HN3M, 7M1HN3M and 7T5M1M gave better results than the inverted sequence, in terms of number of responding mice. 7C1M1M and 7M1C1M gave the same results.

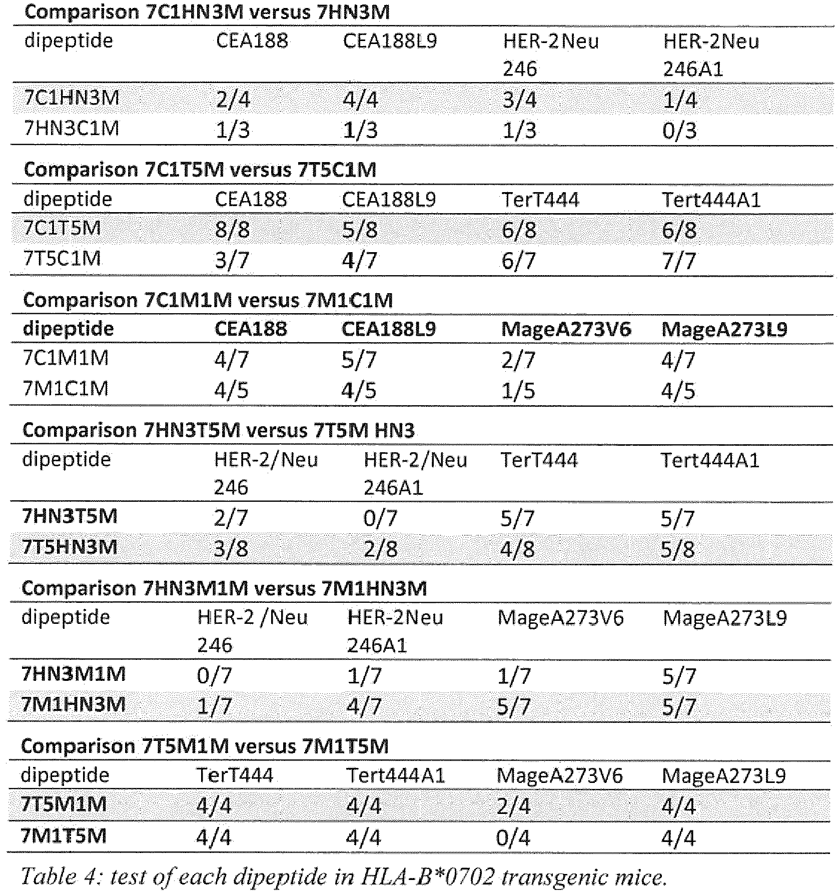

### Example 2: Evaluation of T cell immune response generated in HLA-B*0702 mice after two vaccinations with Vbx-016

To evaluate if the Vbx-016 (SEQ ID No: 23) determined in the dipeptidic experiments is optimum, HLA-B*0702 transgenic mice were vaccinated subcutaneously at the base of the tail with 400µg of Vbx-016 and 150µg of the HBV core T13L helper peptide emulsified in Incomplete Freund Adjuvant (IFA) or Montanide ISA51VG (Seppic, Castres, France) twice at two weeks interval.

After two vaccinations, all mice respond to at least one native peptide, and 13/14 vaccinated mice responded to two or more cognate native peptides. Importantly, each native peptide was recognized at least once in one vaccinated mouse.

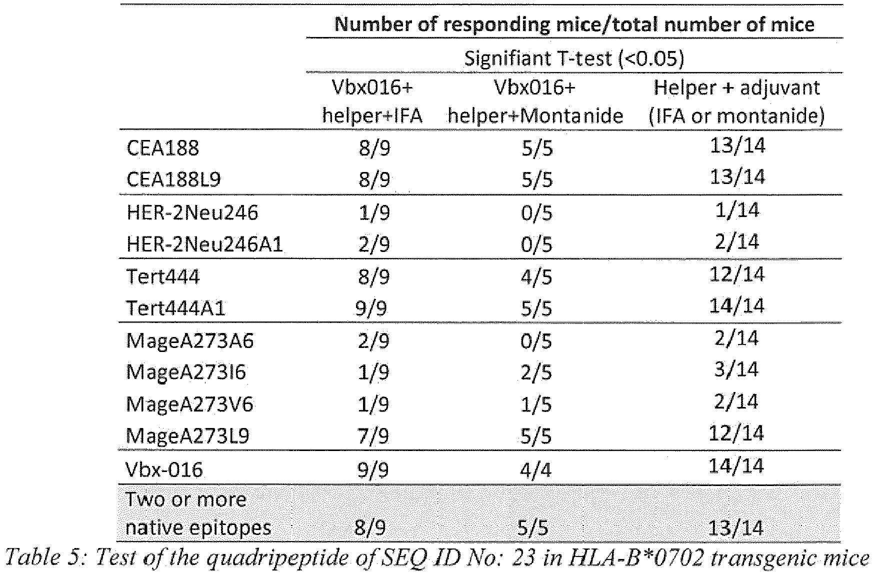

### Example 3: Evaluation of T cell immune response by inducing specific cytotoxic T lymphocytes from human Peripheral Blood Mononuclear Cells in vitro with the dipeptides selected in HLA-B*0702 transgenic mice.

To evaluate if the junction present in the polypeptide CEA_{188L9}/TERT_{444A1}/MAGE_{273L9}/HER-2/neu_{246A1} validated in transgenic mice are processed efficiently in humans, human PBMC were stimulated with each dipeptide (CEA_{188L9}/TERT_{444A1}, TERT_{444A1}/MAGE_{273L9} and MAGE_{273L9}/HER-2/neu_{246A1}) according to the protocol described in the methods. Recognition of the cognate native peptides was evaluated by measuring specific IFNγ producing cells from the CD8+ stimulated by the dipeptide, divided in 4 pools for the test. T test was performed when the mean number of spots obtained for the peptide of interest was superior to the mean number of spots obtained with the irrelevant peptide; when t test value was less than 0.05, the result was considered to be significantly positive and is highlighted in grey in the following table.

In each donor, CTLs were able to recognize both optimized and cognate native peptides (and the four natives for the MAGE-A_{273L9} shared peptide), confirming that each junction is well processed by the proteasome.

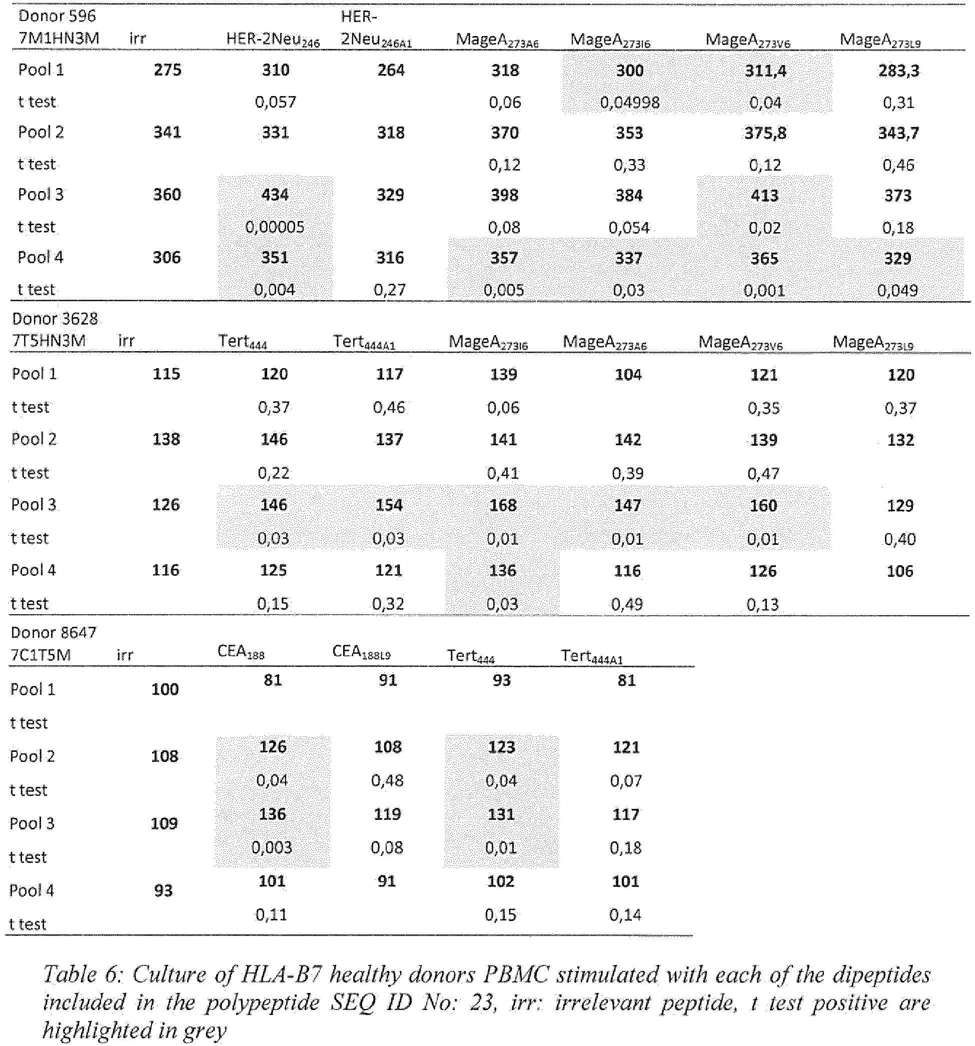

### Example 4: Induction of specific cytotoxic T lymphocytes from human Peripheral Blood Mononuclear Cells in vitro with Vbx-016.

Vbx-016 was finally used to stimulate human PBMC from a healthy donor.

t test was performed when the mean number of spots obtained for the peptide of interest was superior to the mean number of spots obtained with the irrelevant peptide; when t test value was less than 0.05, the result was considered to be significantly positive and is highlighted in grey in the following table. A polyspecific response was induced and several native peptides were recognized by the induced CTLs, confirming that the polypeptide is well processed by the proteasome and that the induced response is polyspecific.

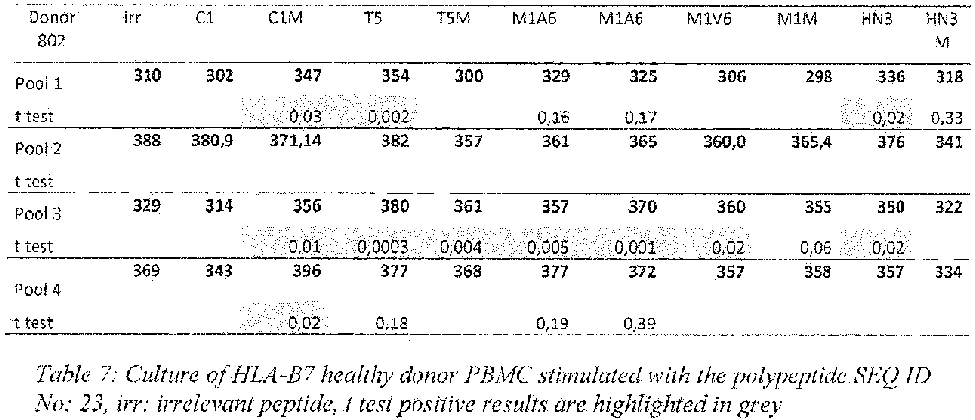

### REFERENCES

Anderson K, Cresswell P, Gammon M, Hermes J, Williamson A, Zweerink H (1991) Endogenously synthesized peptide with an endoplasmic reticulum signal sequence sensitizes antigen processing mutant cells to class I-restricted cell-mediated lysis. J Exp Med 174: 489-492
Babon A, Almunia C, Boccaccio C, Beaumelle B, Gelb MH, Menez A, Maillere B, Abastado JP, Salcedo M, Gillet D (2005) Cross-presentation of a CMV pp65 epitope by human dendritic cells using bee venom PLA2 as a membrane-binding vector. FEBS Lett 579: 1658-1664
Bungener L, Idema J, ter Veer W, Huckriede A, Daemen T, Wilschut J (2002) Virosomes in vaccine development: induction of cytotoxic T lymphocyte activity with virosome-encapsulated protein antigens. JLiposome Res 12: 155-163
Butts C, Murray N, Maksymiuk A, Goss G, Marshall E, Soulieres D, Cormier Y, Ellis P, Price A, Sawhney R, Davis M, Mansi J, Smith C, Vergidis D, Ellis P, MacNeil M, Palmer M (2005) Randomized phase IIB trial of BLP25 liposome vaccine in stage IIIB and IV non-small-cell lung cancer. Journal of clinical oncology : official journal of the American Society of Clinical Oncology 23: 6674-6681
Doling AM, Ballard JD, Shen H, Krishna KM, Ahmed R, Collier RJ, Starnbach MN (1999) Cytotoxic T-lymphocyte epitopes fused to anthrax toxin induce protective antiviral immunity. Infect Immun 67: 3290-3296
Fayolle C, Ladant D, Karimova G, Ullmann A, Leclerc C (1999) Therapy of murine tumors with recombinant Bordetella pertussis adenylate cyclase carrying a cytotoxic T cell epitope. J Immunol 162: 4157-4162
Gross DA, Graff-Dubois S, Opolon P, Comet S, Alves P, Bennaceur-Griscelli A, Faure O, Guillaume P, Firat H, Chouaib S, Lemonnier FA, Davoust J, Miconnet I, Vonderheide RH, Kosmatopoulos K (2004) High vaccination efficiency of low-affinity epitopes in antitumor immunotherapy. J Clin Invest 113: 425-433
Haicheur N, Bismuth E, Bosset S, Adotevi O, Warnier G, Lacabanne V, Regnault A, Desaymard C, Amigorena S, Ricciardi-Castagnoli P, Goud B, Fridman WH, Johannes L, Tartour E (2000) The B subunit of Shiga toxin fused to a tumor antigen elicits CTL and targets dendritic cells to allow MHC class I-restricted presentation of peptides derived from exogenous antigens. J Immunol 165: 3301-3308
Ishioka GY, Fikes J, Hermanson G, Livingston B, Crimi C, Qin M, del Guercio MF, Oseroff C, Dahlberg C, Alexander J, Chesnut RW, Sette A (1999) Utilization of MHC class I transgenic mice for development of minigene DNA vaccines encoding multiple HLA-restricted CTL epitopes. J Immunol 162: 3915-3925
Minev B, Hipp J, Firat H, Schmidt JD, Langlade-Demoyen P, Zanetti M (2000) Cytotoxic T cell immunity against telomerase reverse transcriptase in humans. Proc Natl Acad Sci U S A 97: 4796-4801
Ofuji S, Ikeda M, Tsujitani S, Ikeguchi M, Kaibara N, Yuasa I, Mitsuya K, Katoh M, Ito H (1998) Expression of MAGE-1, MAGE-2 and MAGE-3 genes in human gastric carcinomas; lack of evidence for cytotoxic effects in cases with simultaneous expression of MAGE-3 and HLA-A2. Anticancer Res 18: 3639-3644
Ogata S, Uehara H, Chen A, Itzkowitz SH (1992) Mucin gene expression in colonic tissues and cell lines. Cancer Res 52: 5971-5978
Oukka M, Manuguerra JC, Livaditis N, Tourdot S, Riche N, Vergnon I, Cordopatis P, Kosmatopoulos K (1996) Protection against lethal viral infection by vaccination with nonimmunodominant peptides. JImmunol 157: 3039-3045
Peoples GE, Holmes JP, Hueman MT, Mittendorf EA, Amin A, Khoo S, Dehqanzada ZA, Gurney JM, Woll MM, Ryan GB, Storrer CE, Craig D, Ioannides CG, Ponniah S (2008) Combined clinical trial results of a HER2/neu (E75) vaccine for the prevention of recurrence in high-risk breast cancer patients: U.S. Military Cancer Institute Clinical Trials Group Study I-01 and I-02. Clinical cancer research : an official journal of the American Association for Cancer Research 14: 797-803
Reese DM, Slamon DJ (1997) HER-2/neu signal transduction in human breast and ovarian cancer. Stem Cells 15: 1-8
Rohrlich PS, Cardinaud S, Firat H, Lamari M, Briand P, Escriou N, Lemonnier FA (2003) HLA-B*0702 transgenic, H-2KbDb double-knockout mice: phenotypical and functional characterization in response to influenza virus. Int Immunol 15: 765-772
Rosenberg SA, Yang JC, Restifo NP (2004) Cancer immunotherapy: moving beyond current vaccines. Nat Med 10: 909-915
Slamon DJ, Clark GM, Wong SG, Levin WJ, Ullrich A, McGuire WL (1987) Human breast cancer: correlation of relapse and survival with amplification of the HER-2/neu oncogene. Science 235: 177-182
Van den Eynde BJ, van der Bruggen P (1997) T cell defined tumor antigens. Curr Opin Immunol 9: 684-693
Vonderheide RH, Domchek SM, Schultze JL, George DJ, Hoar KM, Chen DY, Stephans KF, Masutomi K, Loda M, Xia Z, Anderson KS, Hahn WC, Nadler LM (2004) Vaccination of cancer patients against telomerase induces functional antitumor CD8+ T lymphocytes. Clin Cancer Res 10: 828-839
Vonderheide RH, Hahn WC, Schultze JL, Nadler LM (1999) The telomerase catalytic subunit is a widely expressed tumor-associated antigen recognized by cytotoxic T lymphocytes. Immunity 10: 673-679

## Claims

1. A polypeptide **characterized in that** it comprises the sequence SPRLQLSNLXXXAPRRLVQLLXXXGPRALVETLXXXAPKHSDCLA (Seq ID No: 24), wherein the CEA_{188L9} (SEQ ID No: 7), TERT_{444A1} (SEQ ID No: 9), MAGE_{273L9} (SEQ ID No: 10) and HER-2/neu_{246A1} (SEQ **ID** No: 8) epitopes are separated by spacers XXX, in which X is any amino acid or none.

2. The polypeptide according to claim 1, which comprises the sequence SPRLQLSNLAPRRLVQLLGPRALVETLAPKHSDCLA (Seq ID No: 23).

3. The polypeptide according to claim 1 or claim 2, which consists of the sequence SPRLQLSNLAPRRLVQLLGPRALVETLAPKHSDCLA (Seq ID No: 23).

4. The polypeptide according to any of claims 1 to 3, further comprising an endoplasmic reticulum-translocating signal sequence at its N-terminal extremity.

5. The polypeptide according to any of claims 1 to 4, further comprising ubiquitin at its C-terminal extremity.

6. The polypeptide according to any of claims 1 to 5, **characterized in that** it induces a CD8+ T cells response against at least two epitopes selected from the group consisting of CEA₁₈₈, HER-2/neu₂₄₆, TERT₄₄₄, MAGE₂₇₃ A6, MAGE₂₇₃ V6 and MAGE₂₇₃ I6, in a majority of HHD mice vaccinated with said polypeptide.

7. The polypeptide according to any of claims 1 to 6, **characterized in that** it induces a CD8+ T cells response against at least two epitopes selected from the group consisting of CEA₁₈₈, HER-2/neu₂₄₆, TERT₄₄₄, MAGE₂₇₃ A6, MAGE₂₇₃ V6 and MAGE₂₇₃ 16 in an *in vitro* assay with human PBMC from healthy HLA-B*0702 donors.

8. An isolated dendritic cell loaded with a polypeptide according to any of claims 1 to 7.

9. A complex comprising a peptide delivery vector and a polypeptide according to any of claims 1 to 7.

10. A pharmaceutical composition comprising a polypeptide according to any of claims 1 to 7 and/or a dendritic cell according to claim 8 and/or a complex according to claim 9.

11. A polypeptide according to any of claims 1 to 7, and/or a dendritic cell according to claim 8, and/or a complex according to claim 9, for use in cancer immunotherapy in a patient having an HLA- B*0702 phenotype.

12. A kit of parts comprising at least one dose of polypeptide according to any of claims 1 to 7 and at least one dose of adjuvant.

13. A kit of parts comprising at least two doses of polypeptide according to any of claims 1 to 7.

14. The kit of parts according to claim 12 or claim 13, comprising 6 to 20 doses of polypeptide according to any of claims 1 to 7.

15. The kit of parts according to any of claims 12 to 14, wherein each dose of polypeptide comprises between 0.5 and 10 mg of polypeptide.


SEQUENCE LISTING

<110>  VAXON BIOTECH
       GALLON, Catherine
       MENEZ-JAMET, Jeanne

<120>  IMMUNOGENIC POLYPEPTIDE COMPOSED OF HLA-B7 RESTRICTED TUMOR
       ANTIGEN-DERIVED OPTIMIZED CRYPTIC PEPTIDES, AND USES THEREOF

<130>  F1787-9EP_VMAll

<160>  24

<170>  PatentIn version 3.5

<210>  1
<211>  9
<212>  PRT
<213>  Artificial Sequence

<220>
<223>  7C1

<400>  1

Ser Pro Arg Leu Gln Leu Ser Asn Gly
1               5


<210>  2
<211>  9
<212>  PRT
<213>  Artificial Sequence

<220>
<223>  7HN3

<400>  2

Gly Pro Lys His Ser Asp Cys Leu Ala
1               5


<210>  3
<211>  9
<212>  PRT
<213>  Artificial Sequence

<220>
<223>  7T5

<400>  3

Asp Pro Arg Arg Leu Val Gln Leu Leu
1               5


<210>  4
<211>  9
<212>  PRT
<213>  Artificial Sequence

<220>

<223>  7M1A6

<400>  4

Gly Pro Arg Ala Leu Ala Glu Thr Ser
1               5


<210>  5
<211>  9
<212>  PRT
<213>  Artificial Sequence

<220>
<223>  7M1V6

<400>  5

Gly Pro Arg Ala Leu Ile Glu Thr Ser
1               5


<210>  6
<211>  9
<212>  PRT
<213>  Artificial Sequence

<220>
<223>  7M1I6

<400>  6

Gly Pro Arg Ala Leu Val Glu Thr Ser
1               5


<210>  7
<211>  9
<212>  PRT
<213>  Artificial Sequence

<220>
<223>  7C1M

<400>  7

Ser Pro Arg Leu Gln Leu Ser Asn Leu
1               5


<210>  8
<211>  9
<212>  PRT
<213>  Artificial Sequence

<220>
<223>  7HN3M

<400>  8

Ala Pro Lys His Ser Asp Cys Leu Ala
1               5

<210>  9
<211>  9
<212>  PRT
<213>  Artificial Sequence

<220>
<223>  7T5M

<400>  9

Ala Pro Arg Arg Leu Val Gln Leu Leu
1               5


<210>  10
<211>  9
<212>  PRT
<213>  Artificial Sequence

<220>
<223>  7M1M

<400>  10

Gly Pro Arg Ala Leu Val Glu Thr Leu
1               5


<210>  11
<211>  18
<212>  PRT
<213>  Artificial Sequence

<220>
<223>  7C1HN3M

<400>  11

Ser Pro Arg Leu Gln Leu Ser Asn Leu Ala Pro Lys His Ser Asp Cys
1               5                   10                  15


Leu Ala



<210>  12
<211>  18
<212>  PRT
<213>  Artificial Sequence

<220>
<223>  7HN3C1M

<400>  12

Ala Pro Lys His Ser Asp Cys Leu Ala Ser Pro Arg Leu Gln Leu Ser
1               5                   10                  15


Asn Leu

<210>  13
<211>  18
<212>  PRT
<213>  Artificial Sequence

<220>
<223>  7C1T5M

<400>  13

Ser Pro Arg Leu Gln Leu Ser Asn Leu Ala Pro Arg Arg Leu Val Gln
1               5                   10                  15

Leu Leu

<210>  14
<211>  18
<212>  PRT
<213>  Artificial Sequence

<220>
<223>  7T5C1M

<400>  14

Ala Pro Arg Arg Leu Val Gln Leu Leu Ser Pro Arg Leu Gln Leu Ser
1               5                   10                  15

Asn Leu

<210>  15
<211>  18
<212>  PRT
<213>  Artificial Sequence

<220>
<223>  7C1M1M

<400>  15

Ser Pro Arg Leu Gln Leu Ser Asn Leu Gly Pro Arg Ala Leu Val Glu
1               5                   10                  15

Thr Leu

<210>  16
<211>  18
<212>  PRT
<213>  Artificial Sequence

<220>
<223>  7M1C1M

<400>  16

Gly Pro Arg Ala Leu Val Glu Thr Leu Ser Pro Arg Leu Gln Leu Ser
1               5                   10                  15

Asn Leu

<210>  17
<211>  18
<212>  PRT
<213>  Artificial Sequence

<220>
<223>  7HN3T5M

<400>  17

Ala Pro Lys His Ser Asp Cys Leu Ala Ala Pro Arg Arg Leu Val Gln
1               5                   10                  15

Leu Leu

<210>  18
<211>  18
<212>  PRT
<213>  Artificial Sequence

<220>
<223>  7T5HN3M

<400>  18

Ala Pro Arg Arg Leu Val Gln Leu Leu Ala Pro Lys His Ser Asp Cys
1               5                   10                  15

Leu Ala

<210>  19
<211>  18
<212>  PRT
<213>  Artificial Sequence

<220>
<223>  7HN3M1M

<400>  19

Ala Pro Lys His Ser Asp Cys Leu Ala Gly Pro Arg Ala Leu Val Glu
1               5                   10                  15

Thr Leu

<210>  20
<211>  18
<212>  PRT
<213>  Artificial Sequence

<220>
<223>  7M1HN3M

<400>  20

Gly Pro Arg Ala Leu Val Glu Thr Leu Ala Pro Lys His Ser Asp Cys
1               5                   10                  15

Leu Ala

<210>  21
<211>  18
<212>  PRT
<213>  Artificial Sequence

<220>
<223>  7T5M1M

<400>  21

Ala Pro Arg Arg Leu Val Gln Leu Leu Gly Pro Arg Ala Leu Val Glu
1               5                   10                  15

Thr Leu

<210>  22
<211>  18
<212>  PRT
<213>  Artificial Sequence

<220>
<223>  7M1T5M

<400>  22

Gly Pro Arg Ala Leu Val Glu Thr Leu Ala Pro Arg Arg Leu Val Gln
1               5                   10                  15

Leu Leu

<210>  23
<211>  36
<212>  PRT
<213>  Artificial Sequence

<220>
<223>  Polypeptide

<400>  23

Ser Pro Arg Leu Gln Leu Ser Asn Leu Ala Pro Arg Arg Leu Val Gln
1               5                   10                  15

Leu Leu Gly Pro Arg Ala Leu Val Glu Thr Leu Ala Pro Lys His Ser
            20                  25                  30

Asp Cys Leu Ala
        35

<210>  24
<211>  45
<212>  PRT
<213>  Artificial Sequence

<220>
<223>  Polypeptide

<220>
<221>  VARIANT
<222>  (10)..(12)
<223>  X = any amino acid or none

<220>
<221>  VARIANT
<222>  (22)..(24)
<223>  X = any amino acid or none

<220>
<221>  VARIANT
<222>  (31)..(36)
<223>  X = any amino acid or none

<400>  24

Ser Pro Arg Leu Gln Leu Ser Asn Leu Xaa Xaa Xaa Ala Pro Arg Arg
1               5                   10                  15

Leu Val Gln Leu Leu Xaa Xaa Xaa Gly Pro Arg Ala Leu Val Glu Thr
            20                  25                  30

Leu Xaa Xaa Xaa Ala Pro Lys His Ser Asp Cys Leu Ala
        35                  40                  45